# EUROPEAN PATENT APPLICATION

(11) **EP 4 591 714 A1**
(43) Date of publication of application: **30.07.2025**
(21) Application number: 24154024.4
(22) Date of filing: 25.01.2024
(51) Int. Cl.: A23C 19/055, A23C 20/00, A23J 1/00, A23J 3/20, A23L 33/19, C07K 14/47

(54) **ANIMAL-FREE SUBSTITUTE DAIRY FOOD PRODUCTS AND METHODS**

(71) Applicant: Hochland SE, 88178 Heimenkirch (DE)
(72) Inventor: SPIEGEL, Thomas, 88178 Heimenkirch/Allgäu (DE); LEITER, Andreas, 88178 Heimenkirch/Allgäu (DE)
(74) Representative: Patent- und Rechtsanwälte Behrmann Wagner PartG mbB

(57) **Abstract**

The invention relates to a method of producing a substitute dairy food product, the method comprising: preparing an aqueous protein solution comprising a recombinant protein; denaturing the recombinant protein in the aqueous protein solution under conditions that cause an increase in viscosity of the aqueous protein solution while remaining flowable and/or pumpable, wherein the denaturing comprises heating of the aqueous protein solution; addition of a lipid to the aqueous protein solution to achieve a mixture with a fat content of at least 0. 5% w/w; and acidifying the mixture by adding an acidulant and/or bacterial culture, whereby a substitute dairy food product is produced.

## Description

### FIELD OF THE INVENTION

The present disclosure relates to animal-free, or substantially animal-free, substitute dairy food products or dairy analogue food products and methods of making the same. More particularly, the present disclosure relates to methods of making animal-free, or substantially animal-free, substitute dairy food products or dairy analogue food products using recombinant beta-lactoglobulin (rBLG) as a replacement for animal milk proteins.

### BACKGROUND

Prior attempts to make animal-free dairy food products have been met with challenges. Some methods have been limited in the amount of protein that can be added during the making of animal free dairy food products, resulting in end products with limitations on their total protein content. This is mainly due to the fact that plant proteins have a different molecular structure and different functional properties compared to milk proteins. Moreover, some existing methods require homogenization steps and/or shearing treatments, which add complexity and cost to the overall process. Moreover, some existing methods have shown difficulty in controlling unwanted increases in viscosity during the production process, and have also required strict limitations on the presence of any coagulation minerals. Other methods are limited in the types of substitute dairy products that can be made, and the degree to which they sufficiently mimic their animal-based counterparts. Finally, in the absence of caseins, traditional texture building through rennet gelation (cheese) or solely through acid-induced gelation (yoghurt) is not possible, so alternative gelation methods or combinations of these are required.

Taken together, there is an unmet need for the development of commercially viable methods, systems and processes for the preparation of animal-free food products, including animal-free, or substantially animal-free, substitute dairy food products or hybrid substitute dairy food products. The present application fulfills this unmet need, and addresses the existing challenges, by providing a novel method for preparing an animal-free substitute dairy food product using recombinant proteins, as well as a corresponding food product according to the subject-matter of the independent claims. The dependent claims describe advantageous further embodiments of the present invention.

### BRIEF SUMMARY

In one aspect, the invention relates to a method of producing a substitute dairy food product, the method including preparing an aqueous protein solution having a recombinant protein, denaturing the recombinant protein in the aqueous protein solution under conditions that cause an increase in viscosity of the aqueous protein solution, but does not result in formation of a continuous connected solid gel structure, i.e. remains flowable and/or pumpable, where the denaturing includes heating of the aqueous protein solution, addition of a lipid to the aqueous protein solution to achieve a mixture with a fat content of at least 0.5% w/w, and acidifying the mixture by adding an acidulant and/or bacterial culture, whereby a substitute dairy food product is produced.

In some embodiments, such method may also include where the recombinant protein includes a recombinant beta-lactoglobulin. The method may also include where the aqueous protein solution includes about 1% w/w to about 30% w/w protein,. In a further embodiment, the method may also include where the aqueous protein solution includes about 1% w/w to about 50% w/w protein, optionally about 5% w/w to about 45% w/w protein, optionally about 5% w/w to about 40% w/w protein, optionally about 5% w/w to about 35% w/w protein, optionally about 5% w/w to about 30% w/w protein, optionally about 10% w/w to about 30% w/w protein, optionally about 10% w/w to about 25% w/w protein. The method may also include where denaturing the recombinant protein in the aqueous protein solution causes an increase in viscosity of the aqueous protein solution, where the increase in viscosity ranges from about 3-fold to about 1,000-fold.

In some embodiments, the aqueous protein solution may comprise substantially entirely water and recombinant beta-lactoglobulin. In some embodiments, the recombinant protein may be provided as a powder or as a liquid concentrate.

In some embodiments, the aqueous protein solution may have a pH value substantially different from an isoelectric point of the protein. In some embodiments, the pH value is preferably above about pH 6.2. In some embodiments, the pH value is preferably below about pH 3.8.

In some embodiments, the aqueous protein solution may comprise calcium, wherein the calcium content of the aqueous protein solution is below about 0.05% w/w. In some embodiments, the denaturing of the recombinant protein in the aqueous protein solution may comprise heating the aqueous protein solution to about 60°C, about 65°C, about 70°C, about 75°C, about 80°C, about 85°C, about 90°C, about 95°C, about 100°C, about 105°C, about 110°C, about 115°C, or about 120°C.

In some embodiments, the denaturing of the recombinant protein in the aqueous protein solution may comprise heating the aqueous protein solution from about 60°C to about 120°C, optionally from about 65°C to about 110°C, optionally from about 70°C to about 100°C, optionally from about 80°C to about 95°C. In some embodiments, the denaturing of the recombinant protein in the aqueous protein solution may comprise heating the aqueous protein solution for a holding time of about 0.1 seconds to about 1 hour, about 0.1 seconds to about 30 minutes, about 5 seconds to about 10 minutes, about 30 seconds to about 5 minutes, or about 1 minute to about 5 minutes. In some embodiments, the denaturing of the recombinant protein in the aqueous protein solution may comprise heating the aqueous protein solution from about 60°C to about 120°C for a holding time of about 0.1 seconds to about 30 minutes,, optionally wherein a minimum denaturation to achieve a cheese-like texture is dependent on the protein level, optionally wherein at about 10% protein greater than about 90% denaturation is achieved, at about 15% protein greater than about 80% denaturation is achieved, and at about 20% protein greater than about 50% denaturation is achieved. In some embodiments, denaturing the recombinant protein in the aqueous protein solution may cause an increase in viscosity of the aqueous protein solution, optionally wherein the increase in viscosity ranges from about 3-fold to about 1,000-fold, optionally wherein the increase in viscosity ranges from about 5 - 20 mPas for about 10% protein, and about 20 - 2,000 mPas for protein concentrations above about 10% protein, as measured at 20°C and a shear rate of 100 s⁻¹.

In some embodiments, the denaturing may further comprise cooling the aqueous protein solution, optionally wherein the aqueous protein solution is cooled at least to a temperature below the temperature used for denaturing the recombinant protein in the aqueous protein solution (e.g. about 60°C).

In some embodiments, the denaturing further may comprise limited formation of particulate aggregates or microgel particles, for contributing to and/or forming of crumbly texture of the substitute dairy food product, such as for feta, havarti, and cheddar. In some embodiments, the denaturing may be substantially devoid of formation of particulate aggregates or microgel particles resulting in a smooth, elastic texture of the substitute dairy food product, such as for Gouda or Swiss cheeses.

In some embodiments, the lipid may be added to the aqueous protein solution to achieve a fat content of at least about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39% or about 40% w/w. In some embodiments, the lipid may be added to the aqueous protein solution to achieve a fat content in the range of about 0.5% w/w to about 40% w/w, about 1% w/w to about 40% w/w, about 5% w/w to about 35% w/w, about 5% w/w to about 30% w/w, about 10% w/w to about 25% w/w, or about 10% w/w to about 20% w/w. In some embodiments, the lipid added may comprise a vegetable fat, an animal fat, or liquid oil. In some embodiments, the lipid may be a non-animal fat source.

In some embodiments, the method may further comprise emulsification or homogenization of the mixture after adding the lipid. In some embodiments, the lipid may be added to the aqueous protein solution to achieve the mixture prior to the denaturing step.

In some embodiments, the acidifying step may further comprise placing the mixture into a mold. In some embodiments, the acidifying step may further comprise the addition of one or more nutrients (e.g. minerals, vitamins) to allow sufficient bacterial growth and reduction in pH-value. In some embodiments, the acidifying step may further comprise the addition of salt to the mixture, optionally wherein the addition of salt initiates limited aggregation of the denatured recombinant protein, optionally wherein the salt comprises sodium chloride, calcium chloride, or a combination thereof. In some embodiments, the acidifying step may further comprise the addition of an enzyme, optionally a lipase and/or a proteinase, and/or addition of a cheesemaking culture, optionally lactobacilli, lactococci, leuconostoc and/or propionibacteria. In some embodiments, during the acidifying step when a pH value between about pH 6.0 and pH 4.0 is reached, preferably a continuous, solid gel structure is created.

In some embodiments, the substitute dairy food product may comprise a cheese, optionally wherein the cheese comprises a semi-hard and/or hard cheese, e.g. swiss-type, emmental, gouda, tilsit, harvati, or cheddar or cream cheese, cottage cheese, white cheese (feta), soft cheese (brie, camembert), or blue cheese.

In some embodiments, the method may further comprise salting of the substitute dairy food product, optionally wherein the salting comprises a salt bath or salt brine, optionally wherein the substitute dairy food product comprises a cheese. In some embodiments, the salt bath or salt brine may comprise a salt concentration of between about 10% and about 25% w/w and a pH value of between about pH 5 and about pH 6.

In some embodiments, the method may further comprise ripening of the substitute dairy food product at a certain temperature and relative humidity, optionally wherein the temperature ranges from about 2°C to about 30°C, optionally wherein the relative humidity ranges from about 60% to about 99%, optionally about 75% to about 95%.

In some embodiments, the method may further comprise adding one or more nutrients to the substitute dairy food product, optionally wherein the one or more nutrients are added at a concentration sufficient to substantially mimic a nutrient content of an animal-derived dairy food product. In some embodiments, the one or more nutrients may be selected from the group consisting of Calcium, Phosphorous, Vitamin B2, Vitamin B6, Vitamin B12, and Iodine. In some embodiments, the one or more nutrients may be added to the substitute dairy food product at a concentration equal to about 50% to about 100% of a concentration of the same one or more nutrients of an equivalent or substantially equivalent animal-derived dairy food product, optionally at a concentration equal to about 75% to about 100%, about 80% to about 100%, about 85% to about 100%, about 90% to about 100%, about 95% to about 100% or about 99% to about 100% of a concentration of the same one or more nutrients of an equivalent or substantially equivalent animal-derived dairy food product.

In a further aspect, the invention relates to a substitute dairy food product produced by the method according to the application. In some embodiments, the substitute dairy food product may comprise a cheese. In some embodiments, the cheese may be selected from the group consisting of a semi-hard and/or hard cheese (e.g. swiss-type, emmental, gouda, tilsit, harvati, or cheddar), a white cheese (e.g. feta) and a soft cheese (e.g. brie, camembert, blue cheese, cream cheese, cottage cheese). In some embodiments, the substitute dairy food product may include a protein source consisting solely of recombinant protein. In some embodiments, the substitute dairy food product may include a protein source consisting solely of recombinant beta-lactoglobulin. In some embodiments, the substitute dairy food product may be free of animal proteins. In some embodiments, the substitute dairy food product may have a protein concentration of about 10% to about 30% w/w, optionally a protein concentration of at least about 10.0%, at least about 10.5%, at least about 11.0%, at least about 11.5%, at least about 12.0%, at least about 12.5%, at least about 13.0%, at least about 13.5%, at least about 14.0%, at least about 14.5%, at least about 15.0%, at least about 15.5%, at least about 16.0%, at least about 16.5%, at least about 17.0%, at least about 17.5%, at least about 18.0%, at least about 18.5%, at least about 19.0%, at least about 19.5%, at least about 20.0%, at least about 20.5%, at least about 21.0%, at least about 21.5%, at least about 22.0%, at least about 22.5%, at least about 23.0%, at least about 23.5%, at least about 24.0%, at least about 24.5%, at least about 25.0%, at least about 25.5%, at least about 26.0%, at least about 26.5%, at least about 27.0%, at least about 27.5%, at least about 28.0%, at least about 28.5%, at least about 29.0%, at least about 29.5%, and at least about 30.0%. In some embodiments, the substitute dairy food product may comprise about 10% to about 25% recombinant beta-lactoglobulin of which at least 60% is denatured, with about 5% to about 30% of a non-animal lipid, a pH value between about 4.0 and about 6.0, and a hardness between about 15 N and about 120 N. As used herein, "hardness" or "firmness" is defined as a measure of the resistance to deformation, such as an indentation, induced mechanically by pressing. Preferably, hardness or firmness of a substitute dairy food product is measured using a technique as described in Example 11. Hardness or firmness testing loads can be expressed in any suitable unit, as would be appreciated by one of ordinary skill in the art, including for example in Newtons (N).

In some embodiments, the method may further comprise adding one or more components from an animal-derived dairy food product and/or one or more components from an animal-derived milk to form a hybrid substitute dairy food product. In some embodiments, the animal-derived dairy food product and/or the one or more components from an animal-derived milk may comprise caseins, milk minerals, butterfat, dairy cream, and/or dairy cultures.

In a further aspect, the invention relates to a hybrid substitute dairy food product produced by the method according to the invention, optionally wherein the hybrid substitute dairy food product is at least about 50% or more animal-free, or about 50-90% animal-free, or about 60-90% animal-free, or about 70-90% animal-free, or about 80-90% animal-free. In some embodiments, the product may further comprise a hydrocolloid and/or stabilizer configured to support texture and stability (starch, fibers, locust bean gum, guar gum, xanthan, alginate, agar, pectin, cellulose, etc.).

In a further aspect, the invention relates to a substitute dairy food product comprising about 10% to about 30% recombinant beta-lactoglobulin of which at least 60% is denatured, with about 0.5% to about 40% w/w of a non-animal lipid, a pH value between about 4.0 and about 6.0, and preferably a hardness between about 15 N and about 120 N.

Other technical features, details and advantages may be readily apparent to one skilled in the art from the following figures, descriptions, and claims.

### BRIEF DESCRIPTION OF THE DRAWINGS

The features and advantages of the present subject matter will be more readily understood from the following detailed description which should be read in conjunction with the accompanying drawings that are given merely by way of explanatory and non-limiting example, and in which:

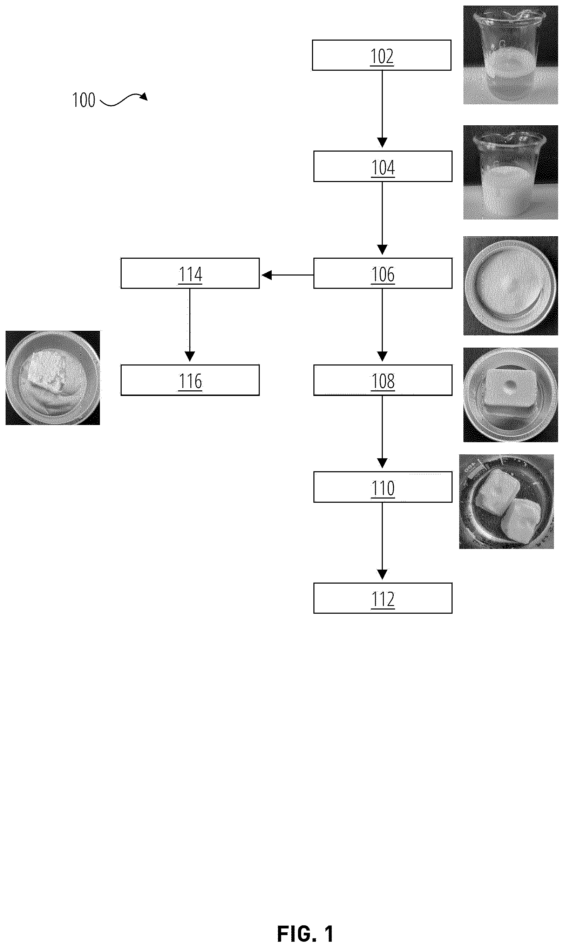

FIG. 1 depicts methods of producing substitute dairy food products as disclosed herein.

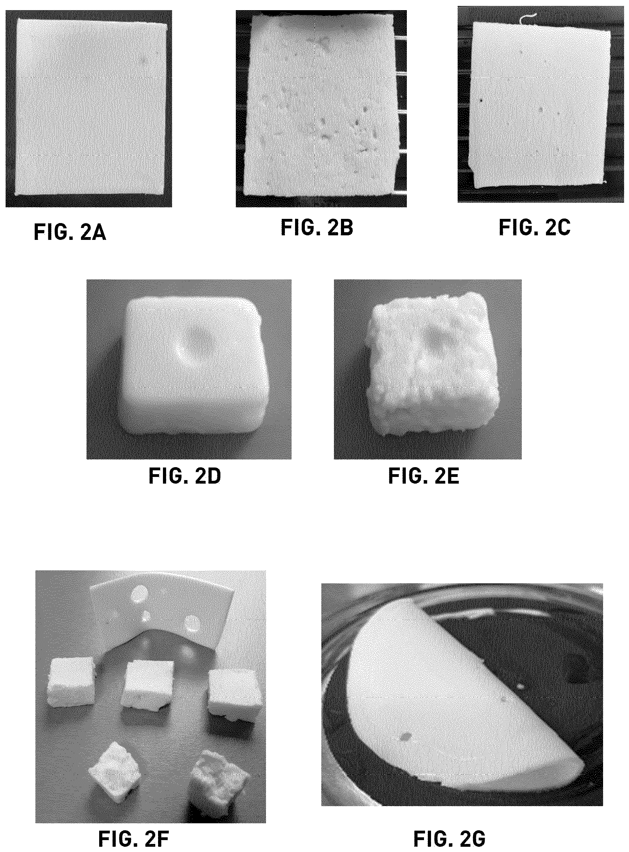

Figs. 2A-2G depict images of prototype cheeses from experiments employing the disclosed methods of making substitute dairy food products based on recombinant beta-lactoglobulin.

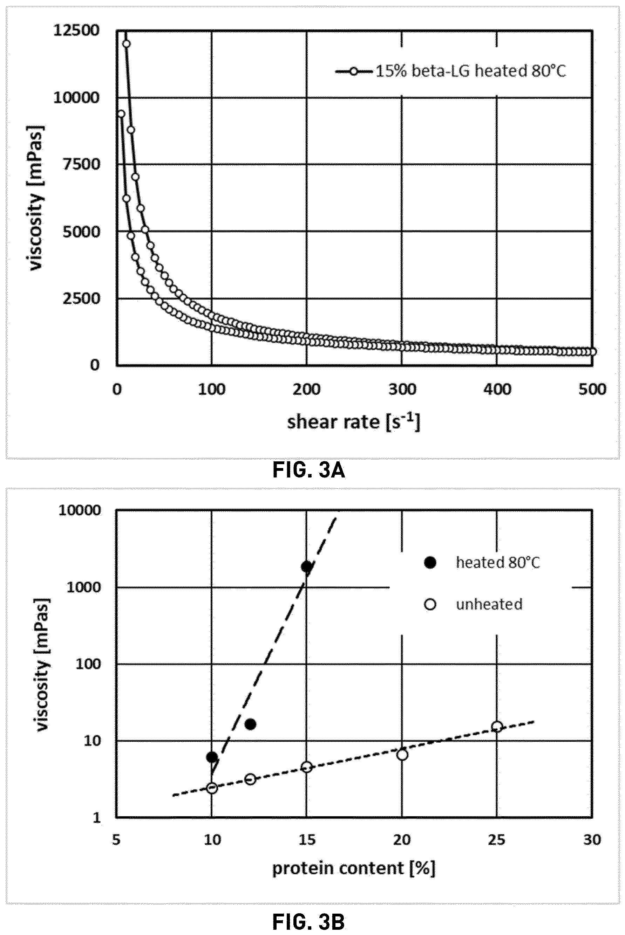

Figs. 3A-3B include data based on testing of the solubility and viscosity of recombinant beta-LG solutions. Fig. 3A is a graphical depiction of viscosity vs. shear rate of heated solution with 15% beta-LG showing pronounced shear thinning properties. Fig. 3B is a graphical depiction of the effect of protein content on viscosity at 20°C of unheated and heated solutions of recombinant beta-lactoglobulin.

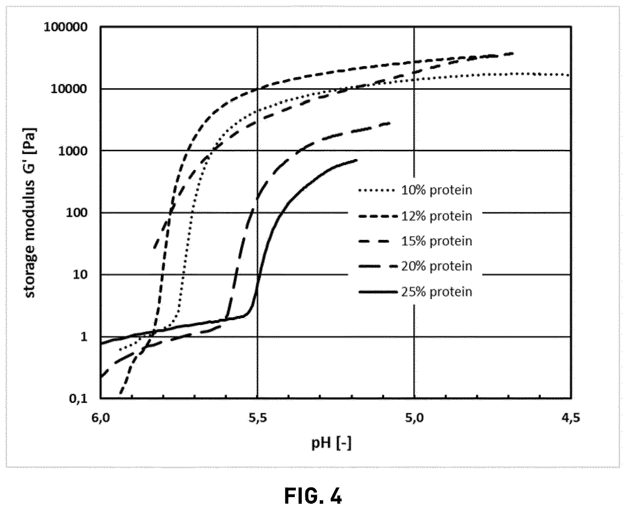

Fig. 4 shows the effect of pH value on storage modulus of beta-LG solutions with various protein contents pre-heated to 75°C for different holding times.

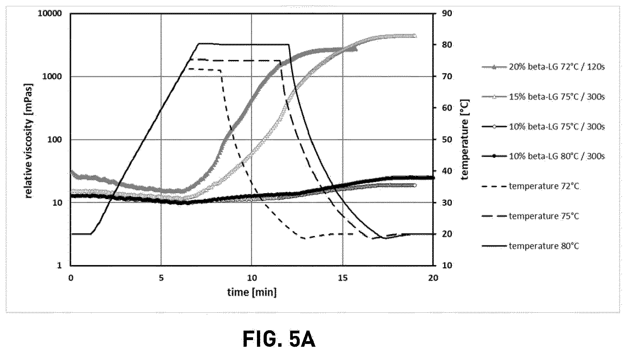

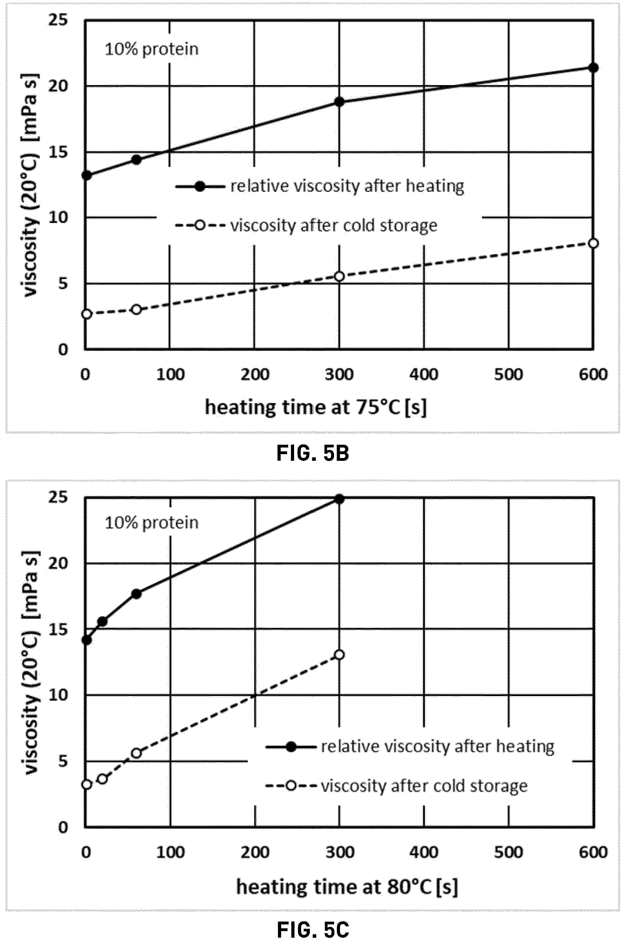

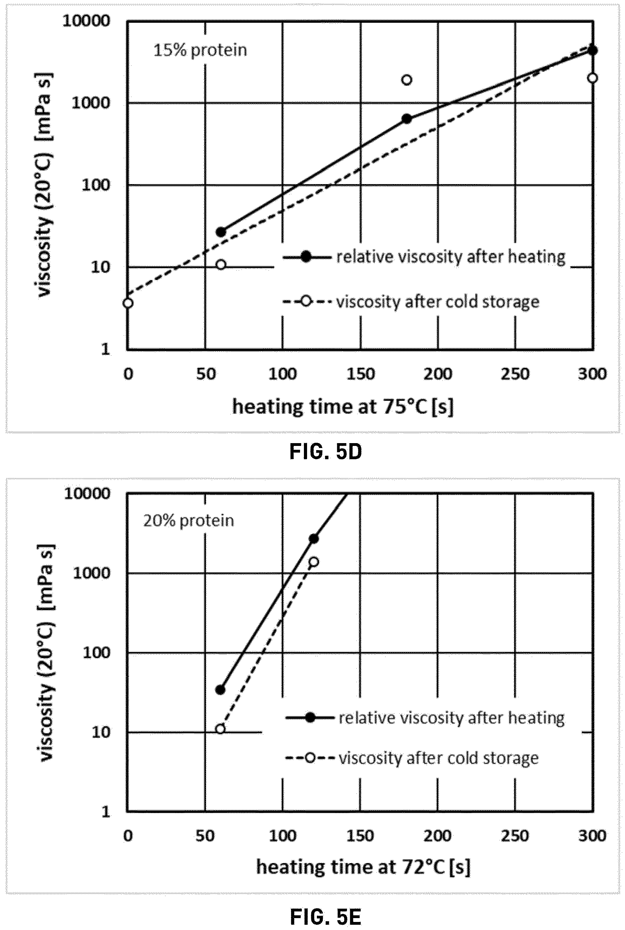

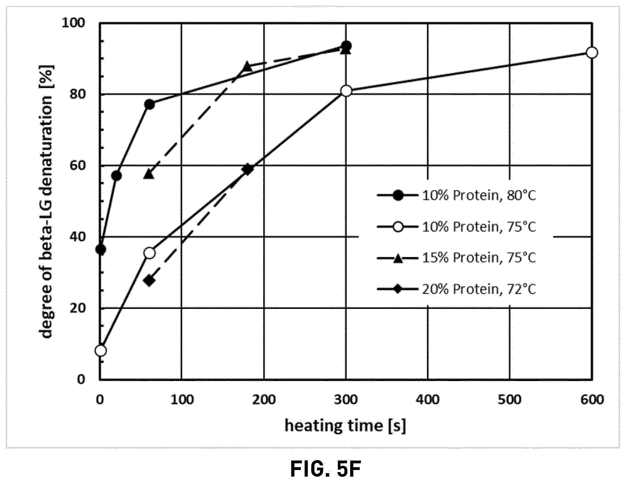

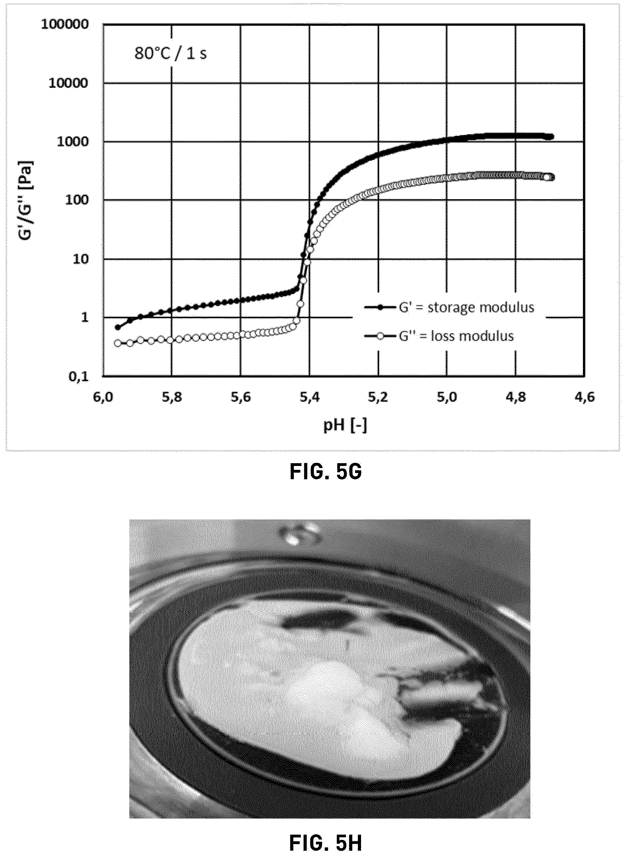

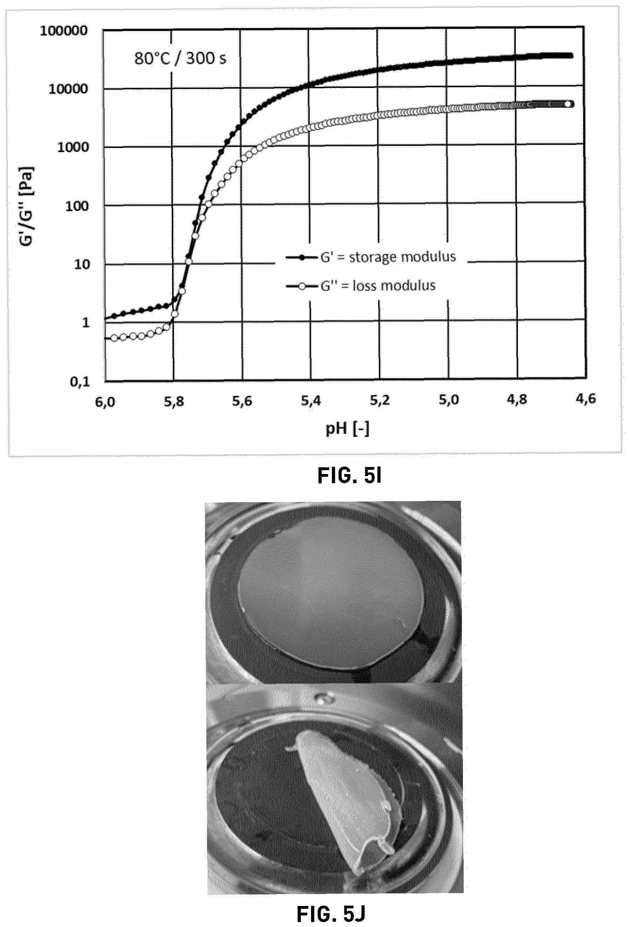

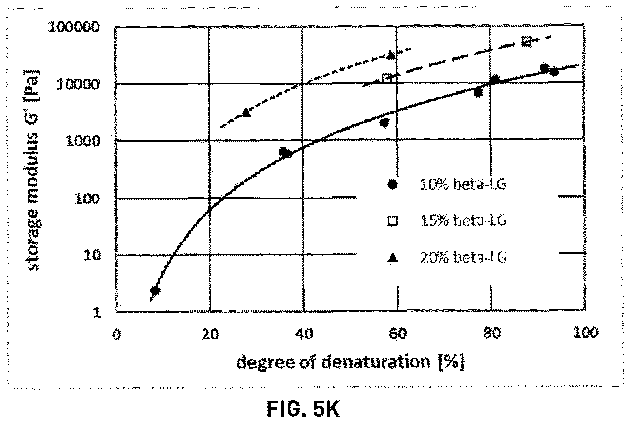

Figs. 5A-5K summarize results of studies evaluating the effect of protein content and heating conditions on beta-lactoglobulin denaturation, viscosity and acid-induced gelation. Fig. 5A shows development of the relative viscosity over time when preheating beta-lactoglobulin solutions for 10% protein (75°C / 300s and 80°C / 300s), 15% protein (75°C / 300s) and 20% protein (72°C / 120s). Fig. 5B is a graphical depiction of the effect of heating time on viscosity for 10% protein solutions heated at 75°C. Fig. 5C shows the effect of heating time on viscosity for 10% protein solutions heated at 80°C. Fig. 5D is a graphical depiction of the data showing the effect of heating time on viscosity for 15% protein solutions heated at 75°C. Fig. 5E illustrates the effect of heating time on viscosity for 20% protein solutions heated at 72°C. Fig. 5F shows the effect of protein content and heating conditions on degree of denaturation of beta-lactoglobulin. Fig. 5G shows the evolution of storage and loss modulus during acidification of 10% beta-LG solutions pre-heated 80°C / 1s. Fig. 5H is an image showing the appearance of 10% beta-LG solutions pre-heated 80°C / 1s on rheometer plate after acidification revealing a homogeneous paste. Fig. 5I shows the evolution of storage and loss modulus during acidification of 10% beta-LG solutions pre-heated 80°C / 300s. Fig. 5J is an image showing the appearance of 10% beta-LG solutions pre-heated 80°C / 300s on rheometer plate after acidification revealing a firm, elastic gel structure. Fig. 5K shows the effect of degree of denaturation on storage modulus G' at pH 5.2 for various protein contents.

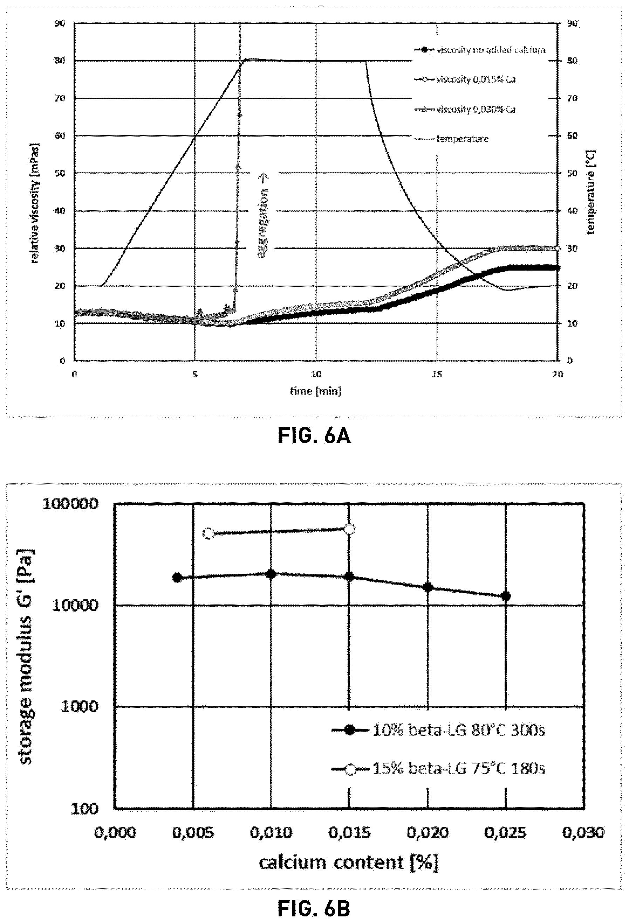

Figs. 6A-6B show the effect of calcium content of the protein solution on beta-lactoglobulin denaturation, viscosity and acid-induced gelation. Fig. 6A shows development of the relative viscosity over time when preheating beta-lactoglobulin solutions with various calcium contents (10% protein, 80°C / 300s). Fig. 6B shows the effect of calcium content on storage modulus G` at pH 5.2.

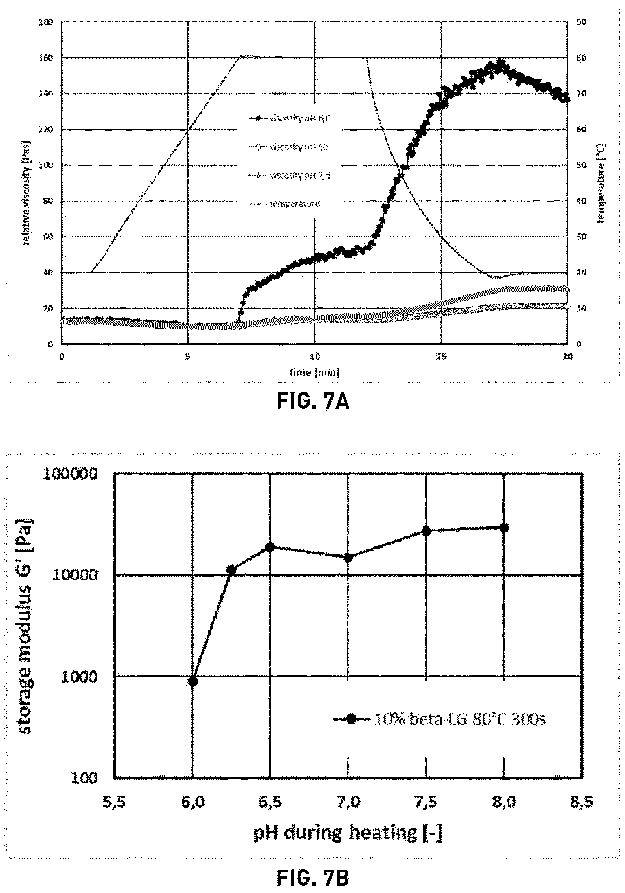

Figs. 7A-7B show the effect of pH-value during heating on beta-lactoglobulin denaturation, viscosity and acid-induced gelation. Fig. 7A shows the development of the relative viscosity overtime when preheating beta-lactoglobulin solutions at different pH values (10% protein, 80°C / 300s). Fig. 7B shows the effect of pH value during heating on storage modulus G` at pH 5.2.

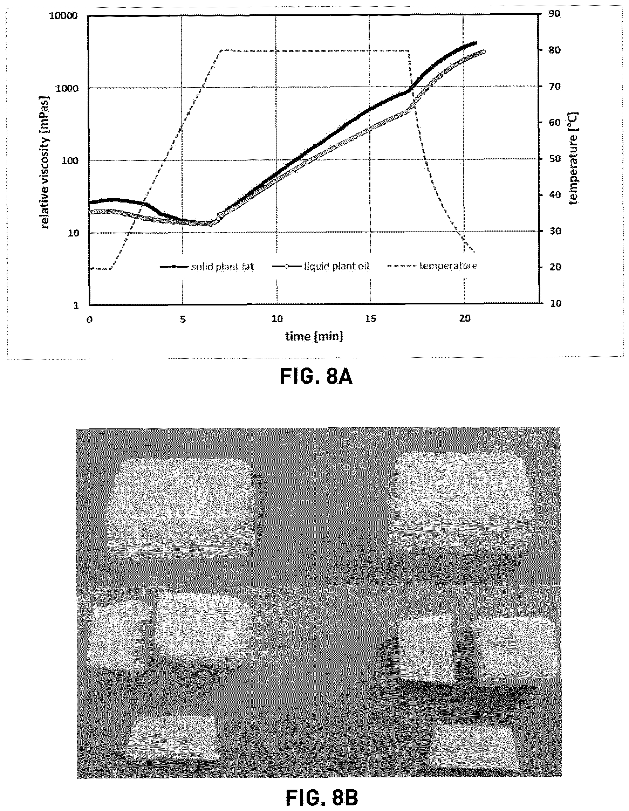

Figs. 8A-8B show the results of emulsions with 10% beta-LG and 22% plant fat or rapeseed oil. Fig. 8A shows the development of the relative viscosity over time when preheating beta-lactoglobulin emulsions with solid plant fat and liquid plant oil. Fig. 8B includes images of emulsion gels prepared with recombinant beta-lactoglobulin and solid plant fat or liquid plant oil.

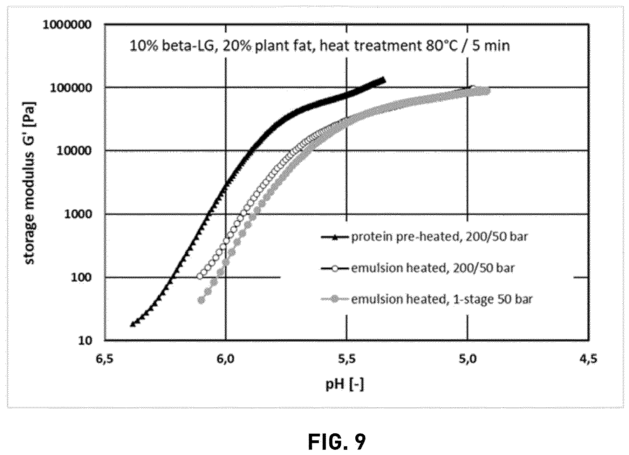

Fig. 9 shows the evolution of storage modulus during acidification of emulsions with 10% beta-LG and 20% plant fat, and the effect of homogenization pressure and order of processing steps heating / emulsification.

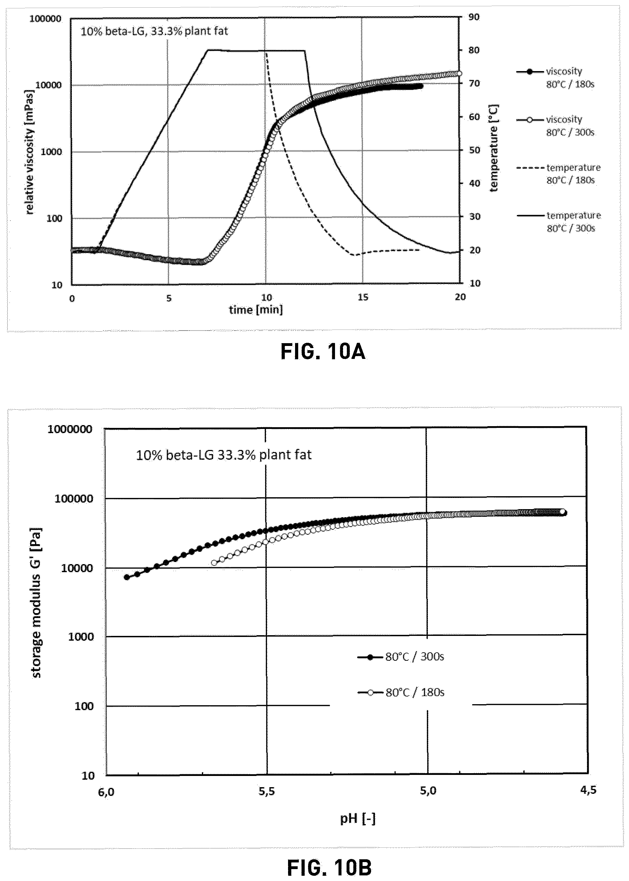

Figs. 10A-10B show the results of analysis of emulsions with 33.3% plant fat. Fig. 10A shows the development of the relative viscosity over time when preheating beta-lactoglobulin emulsions with 33.3% plant fat. Fig. 10B shows the evolution of storage modulus during acidification of emulsions with 10% beta-LG and 33.3% plant fat for different pre-heating times at 80°C.

### DETAILED DESCRIPTION

The presently disclosed subject matter now will be described more fully hereinafter, in which some, but not all embodiments of the presently disclosed subject matter are described. Indeed, the presently disclosed subject matter can be embodied in many different forms and should not be construed as limited to the embodiments set forth herein; rather, these embodiments are provided so that this disclosure will satisfy applicable legal requirements.

### I. Definitions

The terminology used herein is for the purpose of describing particular embodiments only and is not intended to be limiting of the presently disclosed subject matter.

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter.

In describing the presently disclosed subject matter, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the invention and the claims.

Following long-standing patent law convention, the terms "a", "an", and "the" refer to "one or more" when used in this application, including the claims. Thus, for example, reference to "a cell" includes a plurality of such cells, and so forth.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about," when referring to a value or to an amount of a composition, dose, sequence identity (e.g., when comparing two or more nucleotide or amino acid sequences), mass, weight, temperature, time, volume, concentration, percentage, *etc.,* is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed methods or employ the disclosed compositions. In addition, any disclosure in combination with the term "about" is understood as disclosure of at least the respective indicated exact value, amount of a composition, dose, sequence identity, mass, weight, temperature, time, volume, concentration, percentage, etc.

The term "comprising", which is synonymous with "including" "containing" or "characterized by" is inclusive or open-ended and does not exclude additional, unrecited elements or method steps. "Comprising" is a term of art used in claim language which means that the named elements are essential, but other elements can be added and still form a construct within the scope of the claim.

As used herein, the phrase "consisting of' excludes any element, step, or ingredient not specified in the claim. When the phrase "consists of" appears in a clause of the body of a claim, rather than immediately following the preamble, it limits only the element set forth in that clause; other elements are not excluded from the claim as a whole.

As used herein, the phrase "consisting essentially of' limits the scope of a claim to the specified materials or steps, plus those that do not materially affect the basic and novel characteristic(s) of the claimed subject matter.

With respect to the terms "comprising", "consisting of', and "consisting essentially of", where one of these three terms is used herein, the presently disclosed and claimed subject matter can include the use of either of the other two terms.

As used herein, the term "and/or" when used in the context of a listing of entities, refers to the entities being present singly or in combination. Thus, for example, the phrase "A, B, C, and/or D" includes A, B, C, and D individually, but also includes any and all combinations and subcombinations of A, B, C, and D.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The terms "cream cheese", "yogurt", "cheese", "sour cream" and "ice cream" are well known in the art. Such compounds are known as food products made partially and/or substantially from animal milk or component thereof, and can in some embodiments be generally referred to as "dairy food", "dairy foods", "dairy product" or "dairy food products".

As used herein, the terms "dairy food", "dairy product", "edible product", "food product" and the like refer to a product that is suited for human or animal, preferably human, consumption.

As used herein, "Animal-derived dairy food product" refers to dairy foods or dairy food products derived partially, substantially or entirely from an animal product such as milk or milk-components. Animal-derived dairy food product can be used interchangeably with "dairy food product", "dairy food", "dairy" and the like, which are understood in the art as traditional food products derived or made from the milk of a mammalian animal.

As used herein, the terms "substitute dairy food product", "animal-free substitute dairy food product", "dairy substitute", "animal-free dairy substitute", "dairy alternative", "animal-free dairy alternative", "dairy analogue", "animal-free dairy analogue", "dairy analogue food product" and "animal-free dairy analogue food product" are used herein interchangeably and refer to any consumable/edible product or foodstuff, which is not made from or derived from animal milk, i.e. they are substantially or entirely, e.g. about 99.0%, about 99.1%, about 99.2%, about 99.3%, about 99.4%, about 99.5%, about 99.6%, about 99.7%, about 99.8%, about 99.9%, and about 100.0%, free of animal proteins and animal components. Stated another way, the terms "substitute dairy food product", "animal-free substitute dairy food product", and related terms above are intended to include all types of products that contain no milk that comes from cows, goats or other mammals and are devoid of ingredients derived directly from the milk, but yet designed and produced to mimic their corresponding animal-based dairy products. Such products may replace animal-based products in one's diet by attempting to mimic or equal the rheologic and/or organoleptic/physicochemical properties of the traditional animal-milk-based products.

In some embodiments, "hybrid substitute dairy food product" refers to products that comprise combinations of ingredients that are originated from animal milk and those that are not, including in some embodiments where the product is substantially, e.g. 90-99% animal free. In some embodiments, such products can be a combination of animal-derived dairy food product and animal-free substitute dairy food products as defined herein, including for example a product combining animal-derived components and animal-free components resulting in a hybrid substitute dairy food product that is at least about 50% or more animal-free, or about 50-90% animal-free, or about 60-90% animal-free, or about 70-90% animal-free, or about 80-90% animal-free. Such products may replace animal-based products in one's diet by attempting to mimic or equal the rheologic and/or organoleptic/physicochemical properties of the traditional animal-milk-based products.

As used herein, an "Equivalent or substantially equivalent animal-derived dairy food product" refers to a dairy food product derived from an animal for which a substitute dairy food product is intended to mimic or replace.

Such products, including "substitute dairy food product", "animal-free substitute dairy food product", and the like, can include cheese substitutes or cheese analogues, for example, that have at least one property of corresponding animal-based product, e.g. animal-based cheese, including but not limited to appearance, glossiness, consistency, structure, thickness, taste, flavor and penetration strength. According to some embodiments, the substitute dairy food products, including but not limited to cheese analogues, of the present disclosure have 2, 3, 4, 5, 6, 7 or 8 properties of corresponding animal-based dairy products. Such desired properties include but are not limited to: appearance, color, haptics, consistency, texture, firmness, hardness, viscoelastic properties, elasticity, cohesiveness, stickiness, stretch, creaminess, mouth feeling, inner structure (holes, imperfections), meltability, flavor, aroma, and taste.

In the context of the present disclosure, when referring to "non-animal" and "animal-free" products it is to be understood as encompassing a product that is entirely free of animal-derived components, such as BLG or other milk proteins. All proteins of such a product are recombinantly -produced or produced by other synthetic or ex vivo means.

The terms "salt", "coagulation salt", "coagulation mineral" and " coagulation mineral salt" may be herein interchangeably and refer to a salt or mineral, e.g., in the form of soluble salt, or ions thereof that initiates protein coagulation. It is known that soluble salt upon dissolution disintegrates to ions forming it. Solubility of salts may be enhanced during processing e.g. by changes in pH and temperature, so that initially insoluble salts or complexed ions might also be applied to induce targeted coagulation. Thus, according to some embodiments, the term salt refers also to the dissolved salt. Upon dissolution, coagulation mineral salt provides cations that initiate coagulation. According to some embodiments, the coagulation mineral comprises one or more salts of a mineral selected from the group consisting of calcium, sodium (while not as reactive as calcium, sodium can also trigger coagulation e.g. by adding NaCl), magnesium, phosphorus, potassium, selenium, and zinc. In some examples, the coagulation mineral is calcium or magnesium or sodium. In some examples, the coagulation mineral salt is a calcium salt or a magnesium salt or a sodium salt. In some examples, the coagulation mineral salt is selected from the group consisting of calcium chloride, magnesium chloride, and calcium lactate, calcium phosphates, calcium carbonates, calcium citrate, calcium gluconate or any other food-grade calcium salt, or sodium chloride. According to some embodiments, the coagulation mineral salt is calcium chloride, or sodium chloride.

The term "coagulation" or "coagulated" has the meaning of a solidification process in which proteins (and optionally lipids) are made to stick together as a discontinuous phase, thereby becoming separated from their original liquid continuous phase. Typically, denaturation refers to partial unfolding of proteins, e.g. BLG, while aggregation involves very small particles (dimers, octamers), small soluble primary aggregates, as well as large, insoluble particulates. Coagulation refers to interactions between primary aggregates that result in precipitation or formation of a continuous gel network.

The terms "substantially devoid", "essentially devoid", "devoid", "does not include" and "does not comprise" may be used interchangeably and refer to a composition or product that does not include, contain, or comprise a particular component, e.g., the composition comprises less than 0.05 wt%, 0.02 wt%, 0.01 wt%, or less than 0.001 wt% of the component. In some embodiments, the term "devoid" contemplates a composition comprising traces of the devoid component such as traces of a component used in the purification process.

The terms "homogenized", and "homogenization" refer to the process or to the product that passed the process of homogenization. The homogenization may be affected by any known method and device. According to some embodiments, the homogenization is performed in multiple stages, e.g. 1, 2, 3 or 4 stages. According to the principles of the present invention, any homogenization stage and any homogenization pressure found to homogenize the compositions and products of the present invention are included.

The terms "lipid", "fat" and "oil" include any edible lipid. According to some embodiments, the lipid is a non-animal lipid. According to some embodiment, the lipid is plant-derived lipid. In some embodiments where "animal-free" is of less importance an animal fat can be used. According to some embodiments, the edible lipids comprise an oil. According to some embodiment, the oil is selected from the group consisting of shea oil, sunflower oil, coconut oil, rapeseed oil, nut oil, palm oil, kernel oil, olive oil, soya oil, cotton oil, and cocoa butter.

The terms "protein", "protein content", "protein concentration" and "total milk-protein content" refers to the content of all proteins, milk proteins and/or recombinant proteins in the compositions and methods herein, including the resulting substitute dairy food products. In the context of the present disclosure, when referring to "milk protein" it is to be understood as meaning proteins present in milk, yet, not necessarily isolated or derived from milk, i.e. animal-free milk protein substitutes such as rBLG. In certain embodiments, the term "milk protein" refers to BLG, ALA, aS 1 casein, aS2 casein, b casein, and k casein, including recombinantly produced versions thereof.

The term "acidification" refers to the process of reducing the pH of the composition. According to some embodiments, acidification may be affected by any known method. Acidification can be carried out using glucono-delta-lactone (GDL), Lactic Acid Bacteria (LAB), or by using a cheese culture. The LAB bacteria can be a mixed-strain or defined-strain cultures. In some examples, the bacteria culture is mesophilic. In some other examples, the bacteria culture is thermophilic. Typical cultures comprise the species lactococcus, streptococcus, lactobacillus, leuconostoc, bifidobacterium, propionibacterium, brevibacterium Acidification may be carried out by adding an acid, e.g. lactic, citric, acetic, malic acid or any other food grade acidulant.

### II. Methods of making animal-free dairy food substitute food products

The structure of traditional cheese is predominantly formed by the casein network. The whey protein content is generally very low, with the exception of special whey cheeses, e.g. ricotta. Prior to the instant disclosure, it was not expected that a dairy food or substitute thereof could be made using entirely, or substantially entirely, a synthetic or recombinant protein without the benefit of the casein network. Surprisingly, it was discovered and disclosed herein that through a combination of heating, acidification and optionally salting, structures comparable to, for example, feta or semi-hard cheese can be produced based on a recombinant protein, e.g. recombinant beta-lactoglobulin (rBLG), as the sole protein source. By way of example, and not limitation, by using a recombinant beta-lactoglobulin and a vegetable fat, animal-free dairy food substitutes or substitute dairy food products, including for example animal-free cheese substitutes, can be produced in this way.

Because the substitute dairy food products disclosed herein are produced using a controlled method, the proteins, if desired, do not comprise any components obtained from an animal source. Importantly, using the disclosed methods allows for the resulting substitute dairy food products to have the same or substantially the same organoleptic and/or rheologic properties as the dairy food product to which they are analogues, whether by virtue of the production process and/or by the addition of supplemental nutrients during the production process. Therefore, the substitute dairy food products and methods of making the same are intended in some embodiments to substantially mimic their corresponding animal-derived dairy food products.

Embodiments of the methods of producing substitute dairy food products are depicted in Fig. 1. In some embodiments, a method **100** of making a semi-hard cheese substitute dairy food product can include starting with an aqueous whey protein solution **102** (in some embodiments an aqueous whey protein solution that is low in mineral content) followed by step **104** of mixing with fat and emulsifying/homogenizing the solution. Subsequent thereto step **106** can include heating and cooling of the solution as described herein which results in increased viscosity without forming a solid continuous gel, i.e. the result is a flowable and/or pumpable composition. Thereafter, the composition is subjected to an acidification/fermentation step **108** resulting in gelation and/or curd formation, followed by a salting in brine step **110.** Finally, step **112** of ripening yields a semi-hard cheese type substitute dairy food product. In some embodiments, a Feta-type cheese can be formed using a similar method **100,** but with a step **114** comprising direct salt addition to induce limited aggregation coupled with the same acidification/fermentation. Thereafter, ripening **116** results in a Feta-type cheese substitute dairy food product.

Using method **100** of Fig. 1 yielded several prototype substitute dairy food products, including substitute cheese products, examples of which are depicted in Figs. 2A-2G. More particularly, Figs. 2A-2G show exemplary prototypes made from emulsion gels with recombinant BLG and plant fat with different shapes and textures.

Moreover, as discussed further herein, in some embodiments it was surprisingly discovered that the disclosed methods allow for the use of a liquid oil with a high content of unsaturated fatty acids in the making of cheeses and substitute cheese products.

To elaborate, in one aspect, a method of producing a substitute dairy food product, can comprise preparing an aqueous protein solution that includes a recombinant protein, denaturing the recombinant protein in the aqueous protein solution under conditions that cause an increase in viscosity of the aqueous protein solution, but does not result in formation of a continuous connected solid gel structure, where the denaturing includes heating of the aqueous protein solution, addition of a lipid to the aqueous protein solution to achieve a mixture with a fat content of at least 0.5% w/w, and acidifying the mixture by adding an acidulant and/or bacterial culture, whereby a substitute dairy food product is produced. In some aspects, the order of these steps can be rearranged without departing from the scope to the present disclosure. Moreover, the addition of a lipid and/or acidulant/bacterial culture can be optional.

In some embodiments, the recombinant protein can include a recombinant beta-lactoglobulin (rBLG). Recombinant beta-LG is substantially identical with the bovine BLG protein and produced by precision fermentation using genetically modified microorganisms e.g. trichoderma, pichia, or komagataella species. For example, in some embodiments such rBLG can be at least about 75%, 80%, 85%, 90%, 91%, 92%, 93%, 94%, 95%, 96%, 97%, 98%, 99% or more identical (based on the peptide sequence identities) to bovine BLG. By way of example, but not limitation, one such rBLG can be β-lactoglobulin produced by *Komagataella phaffii* strain "yRMK-66"1 (K. phaffii "yRMK-66"). By way of example, but not limitation, one such rBLG can be β-lactoglobulin produced by *Trichoderma reesei* strain QM6a. The method may also include where the aqueous protein solution includes about 1% w/w to about 50% w/w protein, optionally about 5% w/w to about 45% w/w protein, optionally about 5% w/w to about 40% w/w protein, optionally about 5% w/w to about 35% w/w protein, optionally about 5% w/w to about 30% w/w protein, optionally about 10% w/w to about 30% w/w protein, optionally about 10% w/w to about 25% w/w protein, or wherein the aqueous protein solution includes a protein concentration of about 1% w/w, about 2% w/w, about 3% w/w, about 4% w/w, about 5% w/w, about 6% w/w, about 7% w/w, about 8% w/w, about 9% w/w, about 10% w/w, about 11% w/w, about 12% w/w, about 13% w/w, about 14% w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 26% w/w, about 27% w/w, about 28% w/w, about 29% w/w, about 30% w/w, about 31% w/w, about 32% w/w, about 33% w/w, about 34% w/w, about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, and about 50% w/w. The method may also include where the aqueous protein solution includes substantially entirely water and recombinant beta-lactoglobulin. The method may also include where the recombinant protein is provided as a powder or as a liquid concentrate.

In some embodiments, the method may also include where the aqueous protein solution has a pH value substantially different from the isoelectric point of the protein, for example, but without limitation, above about pH 6.2 or below about pH 3.8. The method may also include where the aqueous protein solution includes calcium, where the calcium content of the aqueous protein solution is below about 0.05% w/w, optionally wherein the calcium content ranges from about 0.001% w/w to about 0.05% w/w, preferably in some embodiments about 0.001% w/w to about 0.025% w/w, or wherein the calcium content is about 0.001% w/w, about 0.002% w/w, about 0.003% w/w, about 0.004% w/w, about 0.005% w/w, about 0.006% w/w, about 0.007% w/w, about 0.008% w/w, about 0.009% w/w, about 0.010% w/w, about 0.011% w/w, about 0.012% w/w, about 0.013% w/w, about 0.014% w/w, about 0.015% w/w, about 0.016% w/w, about 0.017% w/w, about 0.018% w/w, about 0.019% w/w, about 0.020% w/w, about 0.021% w/w, about 0.022% w/w, about 0.023% w/w, about 0.024% w/w, about 0.025% w/w, about 0.026% w/w, about 0.027% w/w, about 0.028% w/w, about 0.029% w/w, about 0.030% w/w, about 0.031% w/w, about 0.032% w/w, about 0.033% w/w, about 0.034% w/w, about 0.035% w/w, about 0.036% w/w, about 0.037% w/w, about 0.038% w/w, about 0.039% w/w, about 0.040% w/w, about 0.041% w/w, about 0.042% w/w, about 0.043% w/w, about 0.044% w/w, about 0.045% w/w, about 0.046% w/w, about 0.047% w/w, about 0.048% w/w, about 0.049% w/w, or about 0.050% w/w.

In some embodiments, the calcium content may vary depending on the protein content in the mixture. As discussed further in the Examples and Figures, in some embodiments, the absolute calcium content can be higher at higher protein concentrations. By way of example and not limitation, at 10% protein the Ca limit can be about 0.025% Ca, but 0.030% Ca can be suitable at a protein concentration of 15% or higher. Thus, the Ca:protein ratio can be adjusted higher or lower based on the finding that the absolute calcium content can be higher at higher protein concentrations.

The method may also include where the denaturing of the recombinant protein in the aqueous protein solution includes heating the aqueous protein solution to about about 60°C, about 61°C, about 62°C, about 63°C, about 64°C, about 65°C, about 66°C, about 67°C, about 68°C, about 69°C, about 70°C, about 71°C, about 72°C, about 73°C, about 74°C, about 75°C, about 76°C, about 77°C, about 78°C, about 79°C, about 80°C, about 81°C, about 82°C, about 83°C, about 84°C, about 85°C, about 86°C, about 87°C, about 88°C, about 89°C, about 90°C, about 91°C, about 92°C, about 93°C, about 94°C, about 95°C, about 96°C, about 97°C, about 98°C, about 99°C, about 100°C, about 101°C, about 102°C, about 103°C, about 104°C, about 105°C, about 106°C, about 107°C, about 108°C, about 109°C, about 110°C, about 111°C, about 112°C, about 113°C, about 114°C, about 115°C, about 116°C, about 117°C, about 118°C, about 119°C, or about 120°C. The method may also include where the denaturing of the recombinant protein in the aqueous protein solution includes heating the aqueous protein solution from about 60°C to about 120°C, optionally from about 65°C to about 110°C, optionally from about 70°C to about 100°C, optionally from about 80°C to about 95°C.

The method may also include where the denaturing of the recombinant protein in the aqueous protein solution includes heating the aqueous protein solution for a holding time of about 0.1 seconds to about 1 hour, about 0.1 seconds to about 30 minutes, about 5 seconds to about 10 minutes, about 30 seconds to about 5 minutes, or about 1 minute to about 5 minutes, or about. In some embodiments, the minimum denaturation to achieve a cheese-like texture can be dependent on the protein level. For example, but without limitation, at 10% protein greater than 90% denaturation can be achieved or desired, at 15% protein greater than 80% denaturation can be achieved or desired, and at 20% protein greater than 50% denaturation can be achieved or desired. See also Figs. 5A-5K, 6A-6B and 7A-7B.

In some preferred embodiments, the method may also include where the denaturing of the recombinant protein in the aqueous protein solution includes heating the aqueous protein solution from about 60°C to about 95°C for a holding time of about 0.1 seconds to about 30 minutes.

In some aspects, the method may also include where denaturing the recombinant protein in the aqueous protein solution causes an increase in viscosity of the aqueous protein solution, as compared to the viscosity prior to denaturing, where the increase in viscosity ranges from about a 3-fold increase to about a 1,000-fold increase, optionally an about 3-fold increase, about 4-fold increase, about 5-fold increase, about 10-fold increase, about 15-fold increase, about 20-fold increase, about 25-fold increase, about 30-fold increase, about 35-fold increase, about 40-fold increase, about 45-fold increase, about 50-fold increase, about 55-fold increase, about 60-fold increase, about 65-fold increase, about 70-fold increase, about 75-fold increase, about 80-fold increase, about 85-fold increase, about 90-fold increase, about 95-fold increase, about 100-fold increase, about 125-fold increase, about 150-fold increase, about 175-fold increase, about 200-fold increase, about 225-fold increase, about 250-fold increase, about 275-fold increase, about 300-fold increase, about 325-fold increase, about 350-fold increase, about 375-fold increase, about 400-fold increase, about 425-fold increase, about 450-fold increase, about 475-fold increase, about 500-fold increase, about 525-fold increase, about 550-fold increase, about 575-fold increase, about 600-fold increase, about 625-fold increase, about 650-fold increase, about 675-fold increase, about 700-fold increase, about 725-fold increase, about 750-fold increase, about 775-fold increase, about 800-fold increase, about 825-fold increase, about 850-fold increase, about 875-fold increase, about 900-fold increase, about 925-fold increase, about 950-fold increase, about 975-fold increase, or about 1000-fold increase. Of note, however, the viscosity does not typically increase linearly with increasing protein content and increasing degree of denaturation, but rather exponentially (see Figs. 3B and 5A-5F). By way of example and not limitation, at 10% protein, there may only be a 5-6 fold increase, whereas at 20% protein the increase in viscosity can be up to 1,000-fold after denaturation. The absolute viscosity (measured at 20°C and a shear rate 100 s⁻¹) after heating should be about 5-20 mPas for 10% protein and 20 - 2,000 mPas for higher protein contents. Viscosities above 2,000 mPas are difficult to handle and should be avoided.

The method may also include where the denaturing further includes cooling the aqueous protein solution, optionally where the aqueous protein solution is cooled at least to a temperature below the temperature used for denaturing the recombinant protein in the aqueous protein solution (e.g. about 60°C).

The method may also include where the denaturing further includes limited formation of particulate aggregates or microgel particles, e.g. for formation of cheeses with slightly crumbly textures, such as feta, havarti, or cheddar cheeses. The method may also include where the denaturing is substantially devoid of formation of particulate aggregates or microgel particles, e.g. for formation of cheeses with elastic, smooth textures, such as Gouda or Swiss cheeses. The method may also include where the lipid is added to the aqueous protein solution to achieve a fat content of at least about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39% or about 40% w/w. The method may also include where the lipid is added to the aqueous protein solution to achieve a fat content in the range of about 0.5% w/w to about 40% w/w, about 1% w/w to about 40% w/w, about 5% w/w to about 35% w/w, about 5% w/w to about 30% w/w, about 10% w/w to about 25% w/w, or about 10% w/w to about 20% w/w. The method may also include where the lipid added includes a vegetable fat, an animal fat, or a liquid oil. Nonlimiting examples of suitable lipids include shea oil, sunflower oil, coconut oil, rapeseed oil, nut oil, palm oil, kernel oil, olive oil, soya oil, cotton oil, and cocoa butter. Suitable animal fats can also be used, including for example butter or cream.

The method may also further include emulsification or homogenization of the mixture after adding the lipid. The method may also include where the lipid is added to the aqueous protein solution to achieve the mixture prior to the denaturing step.

In some embodiments, the method may also include where the acidifying step further includes placing the mixture into a mold. The method may also include where the acidifying step further includes the addition of one or more nutrients (e.g. minerals, vitamins) to allow sufficient bacterial growth and reduction in pH-value. In some embodiments, it may be necessary to provide a carbohydrate (e.g. glucose, sucrose, maltose, lactose, maltodextrin) or other specific nutrients (peptides, proteins, minerals, vitamins, trace elements) to allow sufficient bacterial growth and reduction in pH-value, if fermentation is applied. The method may also include where the acidifying step further includes the addition of salt to the mixture, optionally where the addition of salt initiates limited aggregation of the denatured recombinant protein, optionally where the salt includes sodium chloride, calcium chloride, or a combination thereof. The method may also include where the acidifying step further includes the addition of an enzyme, optionally a lipase and/or a proteinase, and/or addition of a cheesemaking culture, optionally lactobacilli, lactococci, leuconostoc and/or propionibacteria. The method may also include where during the acidifying step when a pH value between about pH 6.0 and pH 4.0 (e.g. 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, and 4.0) is reached, a continuous, solid gel structure is created.

The method may also include where the substitute dairy food product includes a cheese, optionally where the cheese includes a semi-hard and/or hard cheese, e.g. swiss-type, emmental, gouda, tilsit, harvati, cheddar, cream cheese, cottage cheese, white cheese (feta), soft cheese (brie, camembert), or blue cheese. The method may also further include salting of the substitute dairy food product, optionally where the salting includes a salt bath or salt brine, optionally where the substitute dairy food product includes a cheese. The method may also further include ripening of the substitute dairy food product at a certain temperature and humidity, optionally where the temperature ranges from about 2°C to about 30°C (e.g. about 2°C, about 3°C, about 4°C, about 5°C, about 6°C, about 7°C, about 8°C, about 9°C, about 10°C, about 11°C, about 12°C, about 13°C, about 14°C, about 15°C, about 16°C, about 17°C, about 18°C, about 19°C, about 20°C, about 21°C, about 22°C, about 23°C, about 24°C, about 25°C, about 26°C, about 27°C, about 28°C, about 29°C, or about 30°C).The humidity, and in particular relative humidity (rH), can range from about 60-99%, preferably about 75-95%. In some embodiments there is also the option of ripening in plastic foil resulting in rindless cheeses.

The method may also further include adding one or more nutrients to the substitute dairy food product, optionally where the one or more nutrients are added at a concentration sufficient to substantially mimic a nutrient content of a corresponding animal-derived dairy food product. By way of example and not limitation, the added or supplemented nutrient can include but is not limited to Calcium, Phosphorous, Vitamin B2, Vitamin B6, Vitamin B12, and Iodine. Additional preservatives, coloring agents or flavoring agents may be added as needed, as would be consistent with traditional dairy food or cheese making. In some embodiments, the one or more nutrients can be added to the substitute dairy food product at a concentration equal to about 50% to about 100% of a concentration of the same one or more nutrients of an equivalent or substantially equivalent animal-derived dairy food product, optionally at a concentration equal to about 75% to about 100%, about 80% to about 100%, about 85% to about 100%, about 90% to about 100%, about 95% to about 100% or about 99% to about 100% of a concentration of the same one or more nutrients of an equivalent or substantially equivalent animal-derived dairy food product.

In some embodiments, the addition of hydrocolloids and/or stabilizers can be included to optionally support texture and stability (starch, fibers, locust bean gum, guar gum, xanthan, alginate, agar, pectin, cellulose etc.).

Provided herein are substitute dairy food product produced by the disclosed methods. By way of example and not limitation, such substitute dairy food products can include cheese, including those selected from the group consisting of a semi-hard and/or hard cheese (e.g. swiss-type, emmental, gouda, tilsit, harvati, or cheddar), a white cheese (e.g. feta) and a soft cheese (e.g. brie, camembert). The substitute dairy food products may also include those with a protein source consisting solely of recombinant protein. The substitute dairy food products in some embodiments include a protein source consisting solely of recombinant beta-lactoglobulin. The substitute dairy food products disclosed herein are considered free or substantially free of animal proteins. Such substitute dairy food products can have a protein concentration of about 10% to about 30% w/w, optionally a protein concentration of at least about 10.0%, at least about 10.5%, at least about 11.0%, at least about 11.5%, at least about 12.0%, at least about 12.5%, at least about 13.0%, at least about 13.5%, at least about 14.0%, at least about 14.5%, at least about 15.0%, at least about 15.5%, at least about 16.0%, at least about 16.5%, at least about 17.0%, at least about 17.5%, at least about 18.0%, at least about 18.5%, at least about 19.0%, at least about 19.5%, at least about 20.0%, at least about 20.5%, at least about 21.0%, at least about 21.5%, at least about 22.0%, at least about 22.5%, at least about 23.0%, at least about 23.5%, at least about 24.0%, at least about 24.5%, at least about 25.0%, at least about 25.5%, at least about 26.0%, at least about 26.5%, at least about 27.0%, at least about 27.5%, at least about 28.0%, at least about 28.5%, at least about 29.0%, at least about 29.5%, and at least about 30.0%. Such high protein levels are surprisingly high as compared to prior animal-free dairy products produced by prior methods unsuitable for such high protein levels.

In some embodiments, the methods disclosed herein may also further include adding one or more components from an animal-derived dairy food product and/or one or more components from an animal-derived milk to form a hybrid substitute dairy food product. Such hybrid products can be advantageous in that they are substantially animal-free but include only specified or select animal-based components thereby avoiding any negative impacts imparted by fully animal-derived products. In such methods of making hybrid substitute dairy food products the method may also include where the animal-derived dairy food product and/or the one or more components from an animal-derived milk includes caseins, milk minerals, butterfat, dairy cream, and/or dairy cultures. In some embodiments, other nutrients and trace elements derived from milk can be added, including for example B-vitamins, bioactive proteins, oligosaccharides or phospholipids. Such hybrid substitute dairy food product produced by these methods are also disclosed herein, where such hybrid products can in some embodiments share all of the same properties as the substitute dairy food products except for the lack of animal-derived components.

By way of example and not limitation, in some embodiments the disclosed methods of making substitute dairy food products can include the following process steps:
1. Preparation of an aqueous protein solution with about 10% w/w to about 30% w/w protein from water and beta-lactoglobulin, including rBLG. The beta-lactoglobulin can be provided as a powder or as a liquid concentrate. In some embodiments, there can also be an optional adjustment of the pH value and mineral content. In some aspects, it can be important that the pH of the solution is far from the isoelectric point of the protein, for example, but without limitation, above pH 6.2 or below pH 3.8, and that the calcium content is relatively low, e.g. below 0.05% w/w (although in some embodiments not devoid of calcium as discussed further herein).
2. Targeted pre-denaturation of the protein solution (in some aspects referred to as a "whey protein solution" as a traditional term of art although no animal whey proteins are present in the present context) by heating to about 60°C to about 95°C for a holding time of from about 0.1 s to about 30 min and cooling. The conditions should generally be chosen so that the viscosity increases, but no solid continuous connected gel structure is formed, i.e. it is still flowable and/or pumpable. For elastic, smooth cheese textures (e.g., Gouda, Swiss Cheese), the formation of particulate aggregates or microgel particles should be avoided completely. However, limited aggregation and gel formation upon heating combined with stirring or shearing can result in slightly crumbly textures reminiscent of feta, havarti, or cheddar cheeses.
3. In some embodiments, there can be an optional addition of a lipid, including but not limited to a vegetable fat or liquid oil, to set a fat content in the range of about 0.5% w/w to about 40% w/w. Emulsification or homogenization of the mixture can also be done as needed. In some embodiments, the order of steps 2 and 3 can be swapped such that the targeted pre-denaturation step comprises heating of a protein stabilized emulsion.

Continuing with step 3 (whether it occurs before or after step 2), it was found by applicants that it is not absolutely necessary to apply high pressures (e.g. by using a dynamic high pressure homogenizer with pressures between 50 and 500 bar) during homogenization to form a fine, stable emulsion with fat droplets smaller than 10 µm. For cheesemaking, it is sufficient to keep the initial emulsion stable until the increase in viscosity during the heating step (if the emulsion is heated) and especially the formation of a homogeneous gel structure during the acidification step prevents the separation of the fat and water phases.

4. Filling the emulsion into molds and acidifying it by directly adding acidulants or bacterial cultures. In some embodiments, it may be necessary to provide certain nutrients (minerals, vitamins) to allow sufficient bacterial growth and reduction in pH-value, if fermentation is applied. In some embodiments, it may be necessary to provide a carbohydrate (e.g. glucose, sucrose, maltose, lactose, maltodextrin) or other specific nutrients (peptides, proteins, minerals, vitamins, trace elements) to allow sufficient bacterial growth and reduction in pH-value, if fermentation is applied. Depending on whether an elastic (e.g. semi-hard cheese) or a slightly crumbly (e.g. feta, havarti) texture is to be achieved, salt (e.g. NaCl or a calcium salt (provided as a concentrated aqueous solution/slurry or as crystalline mineral salt) can be added directly in this step. The addition of salt initiates limited aggregation of the pre-denatured beta-lactoglobulin. Enzymes and special cheesemaking cultures can also be added, which lead to a typical texture and taste profile during ripening, as is known in the art. In addition to the bacteria species mentioned above, white mold (e.g. penicillium camemberti) and blue mold (e.g. penicillium roqueforti) cultures could be added at this stage. Typical enzymes used for flavor development include proteases, peptidases esterases, and lipases. In some embodiments, when a pH value between pH 6.0 and 4.0 is reached, a continuous, solid gel structure can be created.

5. As an optional step, salting of the coagulated cheese base in a salt bath (brine)can in some embodiments be employed. In some embodiments, the brine typically has a salt content (NaCl) between about 10% and 25% w/w and a pH-value between about 5 and 6.

6. Ripening of the cheese at a certain temperature and humidity. If propionibacteria or other gas-producing cultures are added in step 4, the holes (eyes) typical for Swiss-type cheeses will be created during ripening.

Of note, prior attempts to produce substitute dairy food products discovered that when applying a traditional cheese making protocol on an artificially-produced cheese composition comprising BLG as a main milk protein, the composition prematurely coagulates, thus preventing the cheese-analogues product from being produced with the desired properties. See particularly WO2022/239000. Therefore, the methods of WO2022/239000 were limited by what was perceived to be an unwanted increase of viscosity. In marked contrast, and in surprising contradiction to what was shown previously, the presently disclosed methods include a targeted pre-denaturation of the protein solution that allows for, and in some embodiments encourages, an increase in viscosity.

In some embodiments, the presently disclosed methods and resulting substitute dairy products offer advantages over prior method and dairy products based at least in part on an optimized tuning of the heating conditions, pre-denaturation of rBLG, and viscosity increase. Moreover, in some embodiments, it was surprising to find that the disclosed methods allowed for targeted formation of textures that are typically for different types of cheese (smooth, elastic, firm, crumbly). Such formation of textures can be adjusted by pre-denaturation conditions (with or without limited protein aggregation).

As discussed further herein, in some embodiments it was surprising to find that the disclosed methods allow for the production of cheeses and substitute cheese products with a protein content above 10% w/w. For example, and as discussed further herein, the disclosed methods allow for the production of substitute dairy food products with protein contents ranging from 10% w/w up to 30% w/w or more.

Moreover, as discussed further herein, in some embodiments it was surprising to find that the disclosed methods allow for the ripening of cheeses and substitute cheese products with cultures and enzymes optionally resulting in hole formation (e.g. propionibacteria).

### EXAMPLES

The following examples are included to further illustrate various embodiments of the presently disclosed subject matter. However, those of ordinary skill in the art should, in light of the present disclosure, appreciate that many changes can be made in the specific embodiments which are disclosed and still obtain a like or similar result without departing from the spirit and scope of the presently disclosed subject matter.

### Example 1

### Methods for producing prototype cheeses from recombinant beta-lactoglobulin

Using the methods disclosed herein for making substitute dairy food products, including the method depicted in Fig. 1, the first prototypes were produced using a recombinant beta-lactoglobulin and a rapeseed-based vegetable fat or butter. The protein content was set at 10% and the fat content at 20%. Acidification was carried out with GDL (glucono-delta-lactone) at 40°C.
Using the methods disclosed herein, including Fig. 1, prototype cheese products representing substitute dairy food products made from recombinant beta-lactoglobulin, were produced. Exemplary images of such products are shown in Figs. 2A-2G.

### Example 2

### Analytical Methods

### I. Rheological methods Anton Paar Rheometer MCR 302

Experiments were conducted to test the rheological characteristics of substitute dairy food products made from recombinant beta-lactoglobulin as disclosed herein. These experiments included the following:
a) Pre-heating with recording of relative viscosity:
   This method enables a controlled heat treatment (heating, holding, cooling) with simultaneous recording of the relative viscosity. Rheometer starch cell with 6-blade stirrer, filled with 25 g of solution or emulsion, heating rate 20°C / min, stirrer speed 160 rpm.
b) Viscosity at constant shear rate:
   Preheated solutions were stored at least one day at 5°C. Viscosity was measured at 20°C during 2 min stirring at a shear rate of 100 s⁻¹ with a 4° / 40 mm cone and plate system.
c) Flow curves (viscosity vs. shear rate):
   Recording of shear rate ramps from 5 s⁻¹ to 500 s⁻¹ (upwards - holding - downwards, 3 min each) at 20°C using a cone and plate system (4° / 40 mm).
d) Acid gelation and determination of the viscoelastic properties:
   10 g of pre-heated solution were tempered to 40°C. Glucono-delta-Lactone (GDL) was added (dosage 0.7 - 2.0% depending on actual protein content) and stirred in manually with a spatula. Two 2 ml vials were filled and kept at 40°C in a heated aluminium block to visually observe gel formation and record pH-value. Oscillatory rheology was performed simultaneously at 40°C with a 50 mm parallel plate system at a frequency of 1 Hz and a deformation of 0.1%. The method characterizes the viscoelastic properties of the samples and the sol-gel transition during acidification by recording the storage modulus G' and the loss modulus G'' which represent the elastic, solid-like properties and the viscous, liquid-like properties, respectively.

### II. Methods to determine the degree of denaturation:

Heat-treated samples were diluted to 5% protein and adjusted to pH 4.6 with 1 N HCl. Fat and precipitated protein was separated by centrifugation at 12000 g for 20 min in a microcentrifuge. Protein concentration (total protein and acid-soluble protein in aqueous phase) was analyzed with the Dumas method (N x 6.38). As beta-lactoglobulin is the predominant protein in the samples, the degree of denaturation can be calculated from the total protein analysis as follows: Degree of denaturation: DD % = [1 - (soluble protein / total protein)] x 100.

A method to determine total whey protein and acid-soluble (i.e. native) whey protein in matured cheese has been recently described by Oesen et al. (Determination and evaluation of whey protein content in matured cheese via liquid chromatography, Food Science and Technology, 174, 2023). This method can be applied to determine the total beta-lactoglobulin and the denatured beta-lactoglobulin content in the finished substitute dairy food product of the present disclosure.

### Example 3

### Solubility and viscosity of recombinant beta-LG solutions

Recombinant beta-lactoglobulin powder with 95% protein was dissolved in distilled water using a magnetic stirrer to adjust protein concentrations between 10 and 25% (w/w). Protein solutions were filled into 15 ml centrifuge tubes and heated from 20°C to 80°C in a water bath (heating time approx. 7 min) and immediately cooled in ice water. Flow curves were recorded according to Example 2 before and after heat treatment.

The viscosity values were determined using the upward flow curve at a shear rate of 100 s⁻¹. The linear increase in viscosity versus protein content on a logarithmic scale reflects an exponential correlation. See Figs. 3A and 3B.

Observations for unheated solutions:
- rBLG protein powder easily soluble up to at least 25 % protein resembling flowable, clear, slightly yellowish-brownish liquids.
- Exponential increase of viscosity with increasing protein content, however on a low level (Fig. 3B).

Observations for solutions heated to 80°C:
- rBLG solutions with 10 and 12 % protein show only moderate increase in viscosity
- rBLG solutions with 15 % protein show strong increase in viscosity upon heating (about 500-fold compared to unheated solution) resulting in a syrup-like, but still free-flowing consistency, and pronounced shear-thinning behavior (Fig. 3A).
- rBLG solutions with 20% and 25% formed solid gels before reaching 80°C
- Exponential increase of viscosity with increasing protein content (Fig. 3B).

Heated protein solutions with 12% protein or more show pronounced shear-thinning properties, i.e. the viscosity decreases significantly as the shear rate increases. This offers the possibility of managing higher protein contents through constant shear stress (e.g. through stirring) in conjunction with slightly elevated temperatures. Further processing steps (addition of ingredients, incubation with cultures, molding) should follow immediately after heating.

### Example 4

### Acid gelation of pre-heated beta-LG solutions - Screening of suitable pre-heating conditions for protein contents between 10% and 25%

Background: Solutions of beta-lactoglobulin with a low mineral content can be heat-treated to some extent without aggregation and gelation. The formation of a gel structure can be subsequently induced by acidification.

Procedure: Heating of solutions with 10 - 25% beta-LG in 15 ml tubes in a water bath followed by cooling in ice water:
10% 75°C / 45 min
12% 75°C / 30 min
15% 75°C 18 min
20% 75°C / 7 min
25% 75°C / 6 min

Acid induced gelation was conducted according to Example 2 and Figure 4. 1.5% GDL was added for protein levels up to 20% protein and 2.0% GDL was added at 25% protein.

Observations:
- Samples with 10, 12 and 15% protein form firm, elastic gels
- Higher protein levels increase buffering and delay acidification
- Insufficient heat treatment results in a weak gel at 20% protein and a smooth paste at 25% protein. Addition of stabilizers might be expedient to support structure in this case.

### Example 5

### beta-lactoglobulin denaturation, viscosity and acid-induced gelation: Effect of protein content and heating conditions

This set of experiments was conducted to evaluate the effect of protein content and temperature/time combinations of the pre-heating step on protein denaturation, viscosity development and acid-induced gelation.

Protein solution: Recombinant beta-lactoglobulin isolate powder with 95% protein. Solutions of 10%, 15% and 20% w/w in distilled water adjusted to pH7 with 0.1 N NaOH.

### Pre-heating: see Example 2.

Applied trial conditions (protein content, temperature / time combinations,

**Table 1:**

| | | | | | | | | |
|---|---|---|---|---|---|---|---|---|
| 10% Protein | 75°C / 1s | 75°C / 60s | 75°C / 300s | 75°C / 600s | 80°C / 1s | 80°C / 20s | 80°C / 60s | 80°C / 300s |
| 15% Protein | 75°C / 60s | 75°C / 180s | 75°C / 300s | | | | | |
| 20% Protein | 72°C / 60s | 72°C / 120s | 72°C / 180s | | | | | |

### Observations:

Referring to Fig. 5A, viscosity starts to rise at 75°C due to unfolding and denaturation of proteins. After heating for 300s at 80°C, the viscosity of the 10% protein solution at the end of the cooling phase is twice as high as the initial viscosity. At protein contents of 15% and 20%, heat treatment results in a strong increase in viscosity (note logarithmic scale). Viscosity levels of more than 100 times the initial values are achieved. At 20% protein, a solid gel structure formed after 180s at 72°C.

Relative viscosity was determined at the end of the pre-heating step according to Example 2. Standardized viscosity was determined after one day cold storage according to Example 2.

Conclusion (for 10% protein; see Figs. 5B and 5C):
- Moderate increase in viscosity with heating time and heating temperature.

Conclusions (15 and 20% protein; see Figs. 5D and 5E):
- Strong increase in viscosity (up to 500-fold compared to unheated sample) with heating time (note logarithmic scale).
- Faster viscosity increase at 20% protein despite lower heating temperature
- The sample "15% protein heated 75°C / 300ss" was gelled after cold storage.
- The sample "20% protein heated 72°C / 180s" formed a firm gel after cold storage.

Degree of denaturation was determined according to Example 2. Denaturation reaction is accelerated at higher temperatures and higher protein contents. See Fig. 5F. Measured values are in good correlation with kinetic data for whey protein isolate from Wolz (PhD Thesis, 2017).

Acid-induced gelation was performed according to Example 2 with addition of 1.5% GDL. The graphs show the acid-induced gelation indicated by the increase in storage and loss moduli for samples with **10%** beta-lactoglobulin pre-heated at 80°C for 1 s and 300 s as an example. See Figs. 5G-5J.

Fig 5K shows the effect of degree of denaturation on storage modulus G` at pH 5.2 for various protein contents. Higher denaturation degrees result in higher storage modulus (firmer gels). A minimum degree of denaturation is required to achieve elastic and firm gel structure depending on the protein content.

### Example 6

### beta-lactoglobulin denaturation, viscosity and acid-induced gelation Effect of calcium content

This study evaluates the effect of calcium content in the protein solution.

Raw materials: Recombinant beta-lactoglobulin isolate powder with 95% protein. Solutions of 10%, 15% in distilled water adjusted to pH7 with 0.1 N NaOH.

Calcium content was adjusted with a solution of CaCl₂ with 10% calcium w/w.

### Pre-heating: See example 2.

Applied trial conditions (Table 2):

**Table 2.**

| | | | | | |
|---|---|---|---|---|---|
| 10% Protein 80°C / 300s | 0.010% Ca | 0.015% Ca | 0.020 % Ca | 0.025% Ca | 0.030% Ca |
| 15% Protein 80°C / 300s | | 0.015% Ca | | | |

Observations for 10% protein (see Fig 6A):
- Turbidity increases up to about 0.025% calcium without a significant increase in viscosity.
- Strong aggregation and viscosity increase at 0.030%, resulting in the formation of a white, coagulated mass.
- Heat treatment without precipitation possible up to 0.025% Ca.
- No clear effect of calcium on viscosity at 10% protein for the evaluated range of calcium content between 0.004 and 0.025% Ca.

Effect of calcium content on storage modulus G` at pH 5.2 (Fig. 6B):
- There is little change in the storage modulus at pH 5.2 for calcium contents of up to 0.015%. At 10% protein, higher calcium levels lead to a slight reduction of the storage modulus, i.e. to a slight weakening of the gel structure.
- In the evaluated concentration range, the effect of calcium on the degree of denaturation is relatively small (results not shown).

### Example 7

### beta-lactoglobulin denaturation, viscosity and acid-induced gelation Effect of pH-value during heating

This study evaluates the effect of the initial pH-value of the protein solution.

### Materials and methods:

Raw materials: Recombinant beta-lactoglobulin isolate powder with 95% protein. Solutions of 10% in distilled water adjusted in pH with 1 N NaOH or HCl (finetuning with 0.1 N base or acid)

Pre-heating: See Example 2.

Applied trial conditions (Table 3):

**Table 3.**

| | | | | | | |
|---|---|---|---|---|---|---|
| 10% Protein 80°C / 300s | pH 6.0 | pH 6.25 | pH 6.5 | pH 7.0 | pH 7.5 | pH 8.0 |

Fig 7A shows the development of the relative viscosity over time when preheating beta-lactoglobulin solutions at different pH values (10% protein, 80°C / 300s).

Appearance after heating:
- Between pH 7.0 and pH 8.0 the solutions remain clear
- Minimal clouding at pH 6.5
- Milky-white at pH 6.25
- White, coagulated at pH 6.0

Viscosity development:
- Slight increase in relative viscosity with increasing pH in the range between pH 6.25 and pH 8.0.
- The pH 6.0 sample showed a considerable increase in viscosity due to protein aggregation.

Fig. 7B shows the effect of pH value during heating on storage modulus G` at pH 5.2. Similar maximum storage moduli are achieved between pH 6.5 and 8.0 (pH during pre-heating), which results in no effect of pH on final structure in this pH range. Heating at pH 6.25 results in weakening of the acid induced gel structure; aggregated paste is formed with the sample heated at pH 6.0.

### Example 8

### Emulsions with 10% beta-LG and 22% plant fat or rapeseed oil

Mixing Process: weigh distilled water and protein powder into a 200 ml beaker and mix with magnetic stirrer until dissolved. Add melted fat or liquid oil and heat to 60°C with a heatable magnetic stirrer. Emulsify with an Ultra-turrax^{®} disperser at 6,000 rpm for 2 min and let emulsion cool down to 20°C.

Observations as shown in Fig. 8A:
- Viscosity increases gradually after reaching 80°C.
- Further increase in viscosity during cooling.
- Minor differences between solid fat and liquid oil.
- Final relative viscosity (mPas): ca. 4,000 - 5,000 (extrapolated)

Emulsions with liquid plant oil and solid plant fat were evaluated. After heat treatment in rheometer, addition of 0.7% GDL at 40°C (105 mg GDL to 15 g emulsion) - no salt addition at this time. Filling was completed in ice cube tray. Gelation in oven (temperature setting: 42°C) for 1 h and cooling in cold water. Salting in brine for 18 h (15% NaCl, pH 5.4, fridge).

Results (See Fig. 8B): With oil: elastic, cheese-like gel structure, slightly fatty surface, but no visible free fat or syneresis. With solid fat: very similar texture to sample with oil, slightly firmer, slightly brighter white appearance. Firmness is comparable to a traditional semi-hard cheese.

### Example 9

### Emulsions with plant fat- effect of homogenization and order of heating / emulsification

Pre-mixes were prepared in a Thermomix^{®} kitchen cooker with recombinant beta-lactoglobulin powder, tap water and a plant fat (rapeseed blend). A protein solution was prepared first and melted fat was added and mixed at 60°C in a second step. The composition was adjusted to 10% protein and 20% fat.

In one sample, the protein solution was pre-heated to 80°C and held for 5 min in the cooker and cooled down to 60°C before mixing with fat.

Pre-mixes were homogenized in a lab-scale high pressure homogenizer at 200/50 bar (2-stage) or at 50 bar single stage. The median fat droplet size was approx. 1 µm at 200/50 bar and 2 µm at 50 bar.

The viscosity of the emulsions homogenized at 200/50 bar was measured according to Example 2. Pre-heating of the protein solution resulted in an increase in viscosity from 5.4 mPas to 2500 mPas (20°C, 100s⁻¹).

The emulsions prepared with unheated protein were filled in centrifuge tubes (15 ml) and heated in a water bath at 80°C. It took 8 min to reach 80°C in the center of the tube. After 5 min holding time, the samples were immediately cooled in ice water (protein denaturation in presence of fat).

Acid gelation with 1 % GDL at 40°C after pre-heating according to Example 2. After tempering the emulsions to 40°C, 1% GDL was stirred in. Acid gelation was performed according to Example 2.

As shown in Fig. 9, compared to acid gelation of caseins, the texture formation induced by beta-lactoglubulin begins already at a lower pH value. The samples that were heated after emulsification show a similar behavior regardless of the homogenization pressure. Gelation starts below pH 6 and the final pH-value for 1% GDL addition is around 4.9. Storage moduli reach a maximum of almost 100,000 Pa.

The sample with pre-heating of the protein solution prior to emulsification shows a slightly different gelation property. The storage modulus increases already at pH 6.3. The buffering capacity seems to be enhanced, as the final pH is only 5.4. At this pH, a storage modulus of > 100,000 Pa is achieved. The final texture of all emulsion gels appears similar.

Thin, elastic slices with cheese-like texture properties could be prepared with beta-lactoglobulin stabilized emulsions by means of pre-denaturation and acidification at 40°C.

### Example 10

### Emulsions with 33.3% plant fat

These experiments were conducted to evaluate the limits of fat content.

Raw materials: Recombinant beta-lactoglobulin isolate powder with 95% protein. Tap water, plant fat (rapeseed, palm fat blend); adjusted to 10% beta-LG and 33.3 % fat. Pre-heating and acid gelation, see Example 2. Conditions: 80°C / 180s and 80°C 300 s.

As shown in Fig. 10A, strong increases in viscosity after heating and cooling were observed. In some samples there was a mayonnaise-like texture.

As shown in Fig. 10B, soild gels are formed at pH below 5.5. The texture was slightly less elastic compared to samples with 20% fat.

### Example 11

### Emulsions with plant fat- acidification via fermentation with lactic acid bacteria

These experiments were conducted to evaluate fermentation with lactic acid cultures instead of direct acidification with GDL. Glucose as an energy source for bacteria was varied between 1 and 3%. Yeast extract was added to a sample with 3% glucose as an additional nutrient.

### Materials and methods:

Ingredients: Recombinant beta-lactoglobulin isolate powder with 95% protein, glucose, rapeseed blend (native oil / hydrogenated fat), mesophilic cheese culture Danisco MS514 (*Lactococcus lactis ssp lactis, Lactococcus lactis ssp cremoris, Leuconostoc mesenteroides ssp cremoris*)*,* yeast extract powder.

Protein stock solution: A 1000 g batch of a stock solution with 13.33% w/w beta-lactoglobulin was prepared in a Thermomix^{®} cooker using distilled water at 50°C. The concentration was chosen to later dilute the protein with other ingredients to a constant level of 10.0%.

Heat treatment: The protein solution was heated in the Thermomix^{®} for 10 min at 74.7°C ± 0.6 °C and immediately cooled in ice water.

Emulsification: 4 different batches of emulsions (200 g each) were prepared with an Ultra-turrax^{®} disperser (2 min at 16000 rpm). The fat was melted before mixing with the protein solution and the blend was heated to 50 - 60°C before emulsification. Emulsions with 10% beta-LG and 20% plant fat were prepared. 2 % lactic culture was added after emulsification and cooling to 25°C.

Fermentation: The incubated emulsions were filled in 200 ml plastic cups and left for 16 h at room temperature (21-25°C).

Brining: Cut-out cylinders (20 mm diameter, 100 mm height) were optionally salted in a brine (15% NaCl adjusted to pH 5.4 with citric acid) for several hours.

Texture analyzer: To analyze the cheese products, samples were prepared using a cylindrical die cutter with a 20 mm diameter, then trimmed to 10 mm in height. For pre-sliced commercial samples, the die cutter was used to cut samples, which were then stacked to reach 10 mm in height. All samples were kept at 5°C. The cheese disks were analyzed using a TA.XT2 texture analyzer fitted with a 75 mm cylindrical plate. The samples were compressed to 50% of their original height at a crosshead speed of 1.00 mm/s. Hardness (firmness) is defined as the peak force of the compression (Method according to Dobson & Marangoni (Methodology and development of a high-protein plant-based cheese alternative, Current Research in Food Science, 7, 2023).

The results are shown in Table 4.

**Table 4.**

| **sample name** | final pH | hardness without salt [N] | hardness after brining [N] |
|---|---|---|---|
| 1% glucose | 5.29 | 29 | 31 |
| 2% glucose | 5.27 | 26 | 30 |
| 3% glucose | 5.33 | 32 | 35 |
| 3% glucose + yeast extract | 4.65 | 39 | 50 |
| commercial swiss-type cheese | - | - | 43 |
| commercial process cheese | - | - | 52 |

It will be understood that various details of the presently disclosed subject matter may be changed without departing from the scope of the presently disclosed subject matter. Furthermore, the foregoing description is for the purpose of illustration only, and not for the purpose of limitation.

## Claims

1. A method of producing a substitute dairy food product, the method comprising:
preparing an aqueous protein solution comprising a recombinant protein;
denaturing the recombinant protein in the aqueous protein solution under conditions that cause an increase in viscosity of the aqueous protein solution while remaining flowable and/or pumpable, wherein the denaturing comprises heating of the aqueous protein solution;
addition of a lipid to the aqueous protein solution to achieve a mixture with a fat content of at least 0.5% w/w; and
acidifying the mixture by adding an acidulant and/or bacterial culture,
whereby a substitute dairy food product is produced.

2. The method of claim 1, wherein the recombinant protein comprises a recombinant beta-lactoglobulin.

3. The method of claim 1 or 2, wherein the aqueous protein solution comprises about 1% w/w to about 30% w/w protein, preferably about 5% w/w to about 30% w/w protein.

4. The method of any one of claims 1 to 3, wherein the aqueous protein solution has a pH value substantially different from an isoelectric point of the protein, preferably wherein the pH value is above about pH 6.2 and/or wherein the pH value is below about pH 3.8.

5. The method of any one of claims 1 to 4, wherein the aqueous protein solution comprises calcium, wherein the calcium content of the aqueous protein solution is below about 0.05% w/w.

6. The method of any one of claims 1 to 5, wherein the denaturing of the recombinant protein in the aqueous protein solution comprises heating the aqueous protein solution from about 60°C to about 120°C, preferably from about 65°C to about 110°C.

7. The method of any one of claims 1 to 6, wherein the denaturing of the recombinant protein in the aqueous protein solution comprises heating the aqueous protein solution for a holding time of about 0.1 seconds to about 1 hour, preferably of about 0.1 seconds to about 30 minutes.

8. The method of any one of claims 1 to 7, wherein a minimum denaturation to achieve a cheese-like texture is dependent on the protein level, wherein at about 10% protein greater than about 90% denaturation is achieved, at about 15% protein greater than about 80% denaturation is achieved, and at about 20% protein greater than about 50% denaturation is achieved.

9. The method of any one of claims 1 to 8, wherein denaturing the recombinant protein in the aqueous protein solution causes an increase in viscosity of the aqueous protein solution, wherein the increase in viscosity ranges from about 3-fold to about 1,000-fold, preferably wherein the increase in viscosity ranges from about 5 - 20 mPas for about 10% protein, and about 20 - 2,000 mPas for protein concentrations above about 10% protein, as measured at 20°C and a shear rate of 100 s⁻¹.

10. The method of any one of claims 1 to 9, wherein the denaturing further comprises cooling the aqueous protein solution, preferably wherein the aqueous protein solution is cooled at least to a temperature below the temperature used for denaturing the recombinant protein in the aqueous protein solution.

11. The method of any one of claims 1 to 10, wherein the denaturing further comprises limited formation of particulate aggregates or microgel particles for contributing to a crumbly texture of the substitute dairy food product.

12. The method of any one of claims 1 to 10, wherein the denaturing is substantially devoid of formation of particulate aggregates or microgel particles resulting in a smooth, elastic texture of the substitute dairy food product.

13. The method of any one of claims 1 to 12, wherein the lipid is added to the aqueous protein solution to achieve a fat content in the range of about 0.5% w/w to about 40% w/w, preferably about 1% w/w to about 40% w/w, more preferably about 5% w/w to about 35% w/w.

14. The method of any of claims 1 to 13, wherein the lipid is a non-animal fat source.

15. The method of any one of claims 1 to 14, further comprising emulsification or homogenization of the mixture after adding the lipid.

16. The method of any of claims 1 to 15, wherein the lipid is added to the aqueous protein solution to achieve the mixture prior to the denaturing step.

17. The method of any one of claims 1 to 16, wherein the acidifying step further comprises the addition of salt to the mixture, preferably wherein the addition of salt initiates limited aggregation of the denatured recombinant protein, optionally wherein the salt comprises sodium chloride, calcium chloride, or a combination thereof.

18. The method of any one of claims 1 to 17, wherein the acidifying step further comprises the addition of an enzyme, preferably a lipase and/or a proteinase, and/or addition of a cheesemaking culture, preferably lactobacilli, lactococci, leuconostoc and/or propionibacteria.

19. The method of any one of claims 1 to 18, wherein during the acidifying step when a pH value between about pH 6.0 and pH 4.0 is reached, a continuous, solid gel structure is created.

20. The method of any one of claims 1 to 19, wherein the substitute dairy food product comprises a cheese, preferably wherein the cheese comprises a semi-hard and/or hard cheese, such as swiss-type, emmental, gouda, tilsit, harvati, or cheddar or cream cheese, cottage cheese, white cheese (feta), soft cheese (brie, camembert), or blue cheese.

21. The method of any one of claims 1 to 20, further comprising ripening of the substitute dairy food product at a temperature,from about 2°C to about 30°C and a relative humidity, , from about 60% to about 99%, preferably about 75% to about 95%.

22. The method of any one of claims 1 to 21, further comprising adding one or more nutrients to the substitute dairy food product, wherein the one or more nutrients are added at a concentration sufficient to substantially mimic a nutrient content of an animal-derived dairy food product.

23. The method of any one of claims 1 to 22, further comprising adding one or more components from an animal-derived dairy food product and/or one or more components from an animal-derived milk to form a hybrid substitute dairy food product.

24. A substitute dairy food product produced by the method of any one of claims 1 to 23.

25. The substitute dairy food product of claim 24, wherein the substitute dairy food product comprises a cheese, wherein the cheese is preferably selected from the group consisting of a semi-hard and/or hard cheese, such as. swiss-type, emmental, gouda, tilsit, harvati, or cheddar, a white cheese, such as feta, and a soft cheese, such as brie, camembert, blue cheese, cream cheese, cottage cheese.

26. The substitute dairy food product of claim 24 or 25, wherein the substitute dairy food product includes a protein source consisting solely of recombinant protein, preferably recombinant beta-lactoglobulin.

27. The substitute dairy food product of any one of claims 24 to 26, wherein the substitute dairy food product is free of animal proteins.

28. The substitute dairy food product of any one of claims 24 to 27, wherein the substitute dairy food product has a protein concentration of about 10% to about 30% w/w.

29. A substitute dairy food product, wherein the substitute dairy food product comprises about 10% to about 30% w/w recombinant beta-lactoglobulin of which at least 60% is denatured, with about 0.5% to about 40% w/w of a non-animal lipid, a pH value between about 4.0 and about 6.0, and a hardness between about 15 N and about 120 N.
